(19) Europäisches Patentamt
European Patent Office
Office européen des brevets

(11) Numéro de publication: **0 058 100**
**B1**

(12) **FASCICULE DE BREVET EUROPEEN**

(45) Date de publication du fascicule du brevet: **27.12.84**

(21) Numéro de dépôt: **82400064.0**

(22) Date de dépôt: **14.01.82**

(51) Int. Cl.[3]: **C 07 C 47/565,**
C 07 C 47/575, C 07 C 65/30,
C 07 D 317/64, C 07 C 45/38 //
C07C47/58

(54) **Procédé de préparation d'hydroxybenzaldéhydes.**

(30) Priorité: **20.01.81 FR 8100967**

(43) Date de publication de la demande:
**18.08.82 Bulletin 82/33**

(45) Mention de la délivrance du brevet:
**27.12.84 Bulletin 84/52**

(84) Etats contractants désignés:
**BE CH DE FR GB IT LI NL**

(56) Documents cités:
**US-A-3 673 257**

**MONATSHEFTE FÜR CHEMIE, vol. 103, no. 4, 1972, VIENNE, AT P. CLAUS et al.: "Darstellung und Oxydation von Hydroxybenzylalkoholen", pages 1178-1193**

(73) Titulaire: **SOCIETE FRANCAISE HOECHST**
**Société anonyme dite:**
**3, avenue du Général de Gaulle**
**F-92800 Puteaux (FR)**

(72) Inventeur: **Christidis, Yani**
**12, Rue de Constantinople**
**F-75008 Paris (FR)**
Inventeur: **Vallejos, Jean-Claude**
**9, Rue Labat**
**F-75018 Paris (FR)**

(74) Mandataire: **Rinuy, Guy et al**
**Cabinet Rinuy et Santarelli 14, Avenue de la Grande Armée**
**F-75017 Paris (FR)**

**EP 0 058 100 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne un nouveau procédé pour la préparation d'hydroxybenzaldéhydes par oxydation d'alcools primaires hydroxybenzyliques à l'aide d'oxygène ou de gaz contenant de l'oxygène dans des milieux aqueux alcaline.

Dans l'art antérieur, de très nombreux procédés soit chimiques, soit catalytiques, ont été proposés pour oxyder un alcool benzylique en benzaldéhyde correspondant.

On sait, en particulier, oxyder un alcool hydroxybenzylique, substitué ou non, en hydroxybenzaldéhyde correspondant, par oxydation catalytique par l'oxygène ou des gaz contenant de l'oxygène, en miliaux aqueux alcalins et en présence d'un catalyseur métallique à base d'un métal noble comme le platine ou le palladium. Pour éviter des inconvénients propres à ces procédés tels que la nécessité d'utiliser des quantités importantes de catalyseur, la demande de brevet allemand DE AS n° 1.188.069 (US n° 3.321.526) enseigne l'emploi d'acide borique comme activateur des catalyseurs au palladium; d'autres activateurs de ce type d'oxydation ont été proposés comme, par exemple, selon le brevet des Etats-Unis d'Amérique n° 3.673.257, les ions cadmium, cérium, indium, lanthane, cuivre, yttrium, magnésium, uranyle, zinc, cadmiumtétramine ou selon la demande de brevet japonais n° 55—59128, un sel inorganique d'un métal choisi parmi les métaux suivants: plomb, bismuth, argent et étain.

D'autres procédés ont été décrits en vue soit d'abaisser la consommation en métal du groupe du platine, soit d'inhiber des réactions secondaires, soit enfin pour préparer un aldéhyde aromatique particulier.

Ainsi, la demande de brevet français n° 77—13941 (2.350.323) enseigne l'emploi d'un catalyseur à base d'un métal noble du groupe du platine associé à du plomb, de l'argent, du tellure et/ou de l'étain et/ou leurs composés, la demande de brevet français n° 75—09932 (2.305.420) décrit un co-catalyseur à base d'un dérivé du bismuth, la demande de brevet japonais n° 55—22615 révèle un procédé d'oxydation de l'alcool pipéronylique en pipéronal en présence d'un catalyseur à base d'un métal du groupe du platine associé à un sel inorganique d'un métal choisi parmi les métaux suivants: plomb, bismuth, argent et étain et enfin le brevet des Etats-Unis d'Amérique n° 4.190.605 réalise l'accélération de l'oxydation catalytique par activation du catalyseur à base de palladium par réaction préalable de celui-ci avec un alcool hydroxybenzylique.

Il est à signaler que dans tous ces procédés le rôle de la température n'est pas négligeable et peut parfois être déterminant.

Il est aussi connu de réaliser l'oxydation catalytique d'un alcool hydroxybenzylique en présence d'un solvant organique. C'est ainsi que la demands de brevet japonais n° 55—35003 enseigne une telle oxydation conduite soit dans le diméthylsulfoxyde soit dans le diméthylformamide en présence d'un catalyseur au vanadium. De plus la température doit être comprise entre 100 et 180°C.

Ainsi, les procédés d'oxydation d'alcools hydroxybenzyliques en hydroxybenzaldéhydes, en milieu aqueux alcalin, par l'oxygène ou des gaz contenant de l'oxygène, sont des procédés catalytiques demandant un catalyseur métallique constitué soit par un dérivé du vanadium soit par un métal du groups du platine seul ou en association avec divers ions minéraux. Ces catalyseurs onéreux nécessitent, en fin de réaction, des traitements coûteux pour les récupérer et/ou les purifier et entrainent de ce fait des pertes inéluctables grévant d'autant les coûts de fabrication. En outre le respect de températures réactionnelles dépassant souvent 50°C implique des consommations d'énergie thermique non négligeables venant s'ajouter à ces coûts.

Or, il a maintenant été trouvé par la Demanderesse, d'une manière tout à fait inattendue, et c'est l'objet de la présente invention, un nouveau procédé pour la préparation d'hydroxybenzaldéhydes par oxydation d'alcools primaires hydroxybenzyliques à l'aide d'oxygène ou de gaz contenant de l'oxygène, en milieu aqueux alcalin caractérisé par le fait que cette oxydation est effectuée, à une température comprise entre 25 et 50°C.

Suivant une caractéristique de l'invention le milieu aqueux alcalin est constitué par une solution aqueuse d'un hydroxyde alcalin utilisé en une quantité telle que le rapport molaire hydroxyde alcalin/alcool hydroxybenzylique soit compris entre 2,25 et 10. Suivant une variante, le milieu aqueux alcalin peut être additionné d'un solvant organique inerte dans les conditions réactionnelles et miscible à l'eau.

Suivant une autre caractéristique, le milieu réactionnel peut comporter un accélérateur ou un mélange d'accélérateurs à base des métaux suivants: cuivre, cobalt, fer et manganèse.

Plus précisément, selon cette variante du procédé, les accélérateurs susceptibles d'être utilisés sont des ions métalliques issus d'oxydes ou de sels minéraux ou organiques, anhydres ou hydratés, de cuivre, de cobalt, de fer et/ou de manganèse, à divers degrés de valence tels que le sulfate de cuivre II, le chlorure de cobalt II hexahydraté, le chlorure de fer III, le sulfate de manganèse II.

Le procédé selon l'invention offre divers avantage tels que la réalisation de l'oxydation en phase homogène, la suppression de catalyseur à base de métal noble du groupe du platine, la possibilité d'effectuer l'oxydation en absence de catalyseur de quelque nature que ce soit ou selon la variante du procédé en présence d'ions métalliques accélérateurs à divers degrés de valence, peu onéreux, précédemment cités et enfin la possibilité de travailler à des températures qui ne dépassent pas 50°C.

Les quantités d'accélérateur(s) à utiliser selon la variante du procédé selon l'invention peuvent

varier entre de larges limites. L'effet accélérateur est déjà sensible avec des additions de 0,01 équivalent-gramme du métal ou du composé métallique par mole d'alcool hydroxybenzylique engagée. Habituellement, on utilise de 0,01 à 0,5 équivalent-gramme du métal ou du composé métallique par mole d'alcool hydroxybenzylique engagée.

Le procédé selon l'invention est mis en oeuvre en milieu aqueux alcalin, l'agent alcalin étant avantageusement un hydroxyde alcalin tel que l'hydroxyde de sodium ou l'hydroxyde de potassium. On utilise généralement plus d'une mole d'agent alcalin par mole d'alcool hydroxybenzylique mise en oeuvre. Avantageusement, on opère avec un excès égal ou supérieur à 2,25 moles d'agent alcalin par mole d'alcool engagée et le rapport molaire agent alcalin sur alcool engagé est habituellement compris entre 2,25 et 10.

La concentration de l'alcool hydroxybenzylique dans la solution aqueuse alcaline est choisie, en général, de manière à conserver une solution tout au cours de la réaction d'oxydation. Généralement, on adopte une concentration pondérale en alcool hydroxybenzylique comprise entre 5 et 40%. Dans certains cas particuliers où l'alcool de départ présenterait une très mauvaise solubilité en milieu aqueux alcalin, on peut utiliser un tiers solvant organique miscible à l'eau et inerte dans les conditions réactionnelles tel qu'un alcool primaire aliphatique en $C_1$—$C_3$, le dioxanne, de manière à obtenir une solution. On peut facilement déterminer par quelques essais préalables la nature et la quantité de solvant à utiliser dans chaque cas particulier.

Pour réaliser le procédé selon l'invention, il est important que l'oxygène ou les gaz contenant de l'oxygène soient en contact intime avec la solution aqueuse alcaline contenant l'alcool hydroxybenzylique de départ et éventuellement le ou les accélérateur(s). Pour ce faire, on travaille habituellement sous une bonne agitation et sous une pression d'oxygène ou de gaz contenant de l'oxygène supérieure à la pression ambiante.

Avantageusement, on effectue cette oxydation sous une pression comprise entre 2 et 10 bars. On peut, si on le désire, suivre le déroulement de l'oxydation par la quantité d'oxygène absorbée, mais le plus souvent cette absorption s'arrête d'elle même ou se ralentit très fortement lorsque la quantité théorique d'oxygène nécessaire pour former l'aldéhyde cherché a été absorbée.

Mais, préférentiellement, on suit, si nécessaire, l'avancement de la réaction par analyse physique telle que l'analyse par résonance magnétique nucléaire du proton, RMN, d'une prise d'essai préalablement traitée.

On met en oeuvre le procédé selon l'invention à une température comprise entre 25 et 50°C.

Les alcools hydroxybenzyliques primaires utilisés comme produits de départ répondent à la formule générale, I,

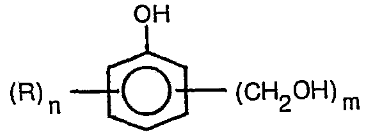

I

dans laquelle

m est égal à 1 ou 2

n est égal à 1, 2, 3,...(5-m)

R, identiques ou différents, représentent un atome d'hydrogène, un atome d'halogène, un groupement alkyle, aryle, alcoxyle, hydroxyle, méthylènedioxy, carboxyle ou un noyau condensé. Les substituante R particulièrement préférés sont des groupements alkyle, alcoxyle, hydroxyle ou méthylènedioxy.

Les alcools hydroxybenzyliques primaires de formule générale, I, sont connus ou peuvent être obtenus selon des moyens connus tels que l'addition d'une ou plusieurs moles de formaldéhyde sur un phénol substitué de formule générale, II:

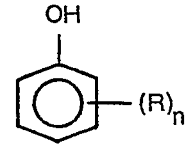

II

dans laquelle

n est égal à 1, 2, 3, 4

et R conserve la signification donnée précédemment.

Un autre avantage du procédé selon l'invention est qu'il peut être mis en oeuvre au départ d'alcools hydroxybenzyliques bruts, non purifiés, contenant entre autres diverses impuretés issues de leurs préparations, ou encore au départ de mélanges d'alcool hydroxybenzyliques isomères (isomères de constitution). Dans ce dernier cas, en fin de réaction d'oxydation, on purifie les hydroxybenzaldéhydes formés selon des moyens connus tels que l'entrainement à la vapeur d'eau, la distillation, la cristallisation fractionnée, la chromatographie.

On peut aussi oxyder sélectivement un des deux groupements hydroxyméthyle en aldehyde; on obtient ainsi des hydroxybenzaldéhydes substitués, entre autres, par un groupement hydroxyméthyle.

En fin de réaction, l'isolement de l'hydroxybenzaldéhyde cherché est réalisé par acidification à pH inférieur ou égal à 3,5 du milieu réactionnel préalablement filtré, si besoin est, pour éliminer éventuellement quelques impuretés mécaniques, suivie d'une séparation de l'hydroxybenzaldéhyde ainsi libéré selon des moyens connus en soi tels que filtration, extraction, entrainement à la vapeur d'eau. Si nécessaire, l'hydroxybenzaldéhyde peut être ensuite purifié par des moyens connus tels que recristallisation, distillation, etc.

Les temps de réaction varient en fonction de nombreux facteurs: nature de l'alcool de départ, vitesse d'agitation, température réactionnelle, pression d'oxygène ou de gaz contenant de l'oxygène, concentration en accélérateur et en agent alcalin, etc...et ils peuvent varier dans une large gamme, mais ils sont en général compris entre 1 heure et 48 heures.

Les hydroxybenzaldéhydes obtenus selon le procédé de l'invention sont d'importants intermédiaires de synthèse organique et/ou des matières précieuses pour l'obtention d'arômes ou de parfums.

Les exemples suivants sont destinés à illustrer le procédé selon l'invention, mais ne le limitent en aucune façon.

Exemples 1 à 3:

On chauffe à 50°C durant 24 heures sous agitation de 1000 tours par minute et sous une pression d'oxygène de P bars, une solution de:

— 0,63 mole (78,2 g) d'alcool parahydroxybenzylique, AHB,
— a moles (y g) d'hydroxyde de sodium en pastilles, dans 300 g d'eau.

En fin de réaction, le milieu réactionnel refroidi et remené à la pression ambiante est dilué à l'eau, puis acidifié à pH=3 avec de l'acide chlorhydrique concentré, filtré, si nécessaire, pour éliminer des impuretés mécaniques et enfin soumis à des extractions répétées avec de l'acétate d'éthyle. Les phases organiques d'extraction réunies sont ensuite lavées à l'eau, séchées sur sulfate de magnésium anhydre, filtrées et concentrées à sec sous vide à une température inférieure ou égale à 30°C. On isole ainsi b grammes d'un mélange d'alcool de départ et de parahydroxybenzaldéhyde, PHB, sous forme d'huile, dont les proportions relatives sont déterminées par résonance magnétique nucléaire, RMN, en solution dans le chloroforme deutéré. Dans le tableau I suivant, sont mentionnés les différents facteurs de ces exemples ainsi que les rendements, R, les taux de conversion, T, et les sélectivités, S. Le rendement, R, est le nombre de moles d'aldéhyde formé sur le nombre de moles d'alcool mis en oeuvre, multiplié par cent. Le taux de conversion, T, est le rapport du nombre de moles d'alcool consommé sur le nombre de moles d'alcool mis en oeuvre, multiplié par cent. La sélectivité, S, est le rapport du nombre de moles d'aldéhyde formé sur le nombre de moles d'alcool consommé, multiplié par cent. Ces trois paramètres sont reliés par la relation:

$$R = S.T.\frac{1}{100}$$

TABLEAU I

| Ex. n° | Na moles a | OH grammes y | Rapport molaire NaOH/alcool | Pression $O_2$ en bars P | Poids brut en $g_b$ | % massiques aldéhyde PHB | % massiques alcool AHB | S% | T% | R% |
|---|---|---|---|---|---|---|---|---|---|---|
| 1 | 2,88 | 115,2 | 4,6 | 8 | 34,5 | 61,9 | 38,1 | 33,4 | 83 | 27,8 |
| 2 | 1 | 40 | 1,6 | 8 | 59,4 | 10 | 90 | 24,5 | 31,7 | 7,8 |
| 3 | 5,76 | 230,4 | 9,1 | 8 | 72,5 | 57 | 43 | 88,9 | 60,3 | 53,6 |

Exemples 4 à 8:

On chauffe à 50°C durant 24 heures sous une agitation de 1000 tours par minute et sous une pression de 8 bars d'oxygène un mélange de:

- 0,63 mole (78,2 g) d'alcool parahydroxybenzylique,
- 2,88 moles (115,2 g) d'hydroxyde de sodium en pastilles,
- c moles (x grammes) d'un sel métallique choisi parmi les sels suivants: sulfate cuivrique pentahydraté, chlorure de cobalt II hexahydraté, chlorure ferrique hexahydraté, sulfate de nickel II heptahydraté, sulfate de manganèse II tétrahydraté dans 300 g d'eau. On opère ensuite selon l'exemple 1. Les résultats obtenus sont donnés dans le tableau II. Le rapport molaire sel métallique sur alcool parahydroxybenzylique est de 0,0127.

TABLEAU II

| Ex. n° | Sel métallique moles | Sel métallique poids en g | Nature du sel métallique | Rapport molaire sel métallique sur alcool | Poids du produit brut en g b | % massiques aldéhyde PHB | % massiques alcool AHB | S% | T% | R% |
|---|---|---|---|---|---|---|---|---|---|---|
| 4 | 0,008 | 2 | $CuSO_4,5H_2O$ | 0,0127 | 69,3 | 82,3 | 17,7 | 88,1 | 84 | 74,1 |
| 5 | 0,008 | 1,9 | $CoCl_2,6H_2O$ | 0,0127 | 64,4 | 100 | 0 | 83,8 | 100 | 83,8 |
| 6 | 0,008 | 2,2 | $FeCl_3,6H_2O$ | 0,0127 | 49,6 | 51,8 | 48,2 | 47,7 | 69,8 | 33,4 |
| 7 | 0,008 | 2,24 | $NiSO_4,7H_2O$ | 0,0127 | 34,2 | 56 | 44 | 30,8 | 80,9 | 24,9 |
| 8 | 0,008 | 1,8 | $MnSO_4,4H_2O$ | 0,0127 | 57,9 | 46,6 | 53,4 | 57,9 | 60,3 | 34,9 |

Exemples 9 à 16:

On chauffe à 8°C durant t heures sous une agitation de 1000 tours par minute et sous une pression de P bars d'oxygène, une solution de

— 0,63 mole (78,2 g) d'alcool parahydroxybenzylique
— a moles (y g) d'hydroxyde de sodium en pastilles
— c moles (z g) de sulfate cuivrique pentahydraté dans n grammes d'eau.

On opère ensuite selon l'exemple 1. Les résultate obtenus sont donnés dans le tableau III.

TABLEAU III

| Ex n° | NaOH a moles | NaOH y g | Eau n en g | $CuSO_4,5H_2O$ moles c | $CuSO_4,5H_2O$ g z | Tempé-rature 8°C | Durée t en h | Pression 02 en bars P | Poids de produit brut en grammes b | % massiques aldéhyde PHB | % massiques alcool AHB | S% | T% | R% |
|---|---|---|---|---|---|---|---|---|---|---|---|---|---|---|
| 9 | 1,62 | 64,8 | 300 | 0,046 | 11,4 | 50 | 8 | 8 | 49 | 94 | 6 | 62,2 | 96,2 | 59,8 |
| 10 | 2,88 | 115,2 | 300 | 0,046 | 11,4 | 50 | 24 | 8 | 74,7 | 100 | 0 | 97,7 | 100 | 97,7 |
| 11 | 1,62 | 64,8 | 300 | 0,008 | 2 | 50 | 24 | 8 | 69 | 100 | 0 | 89,8 | 100 | 89,8 |
| 12 | 1,62 | 64,8 | 300 | 0,046 | 11,4 | 50 | 8 | 2 | 73,5 | 92 | 8 | 94,3 | 92,5 | 87,3 |
| 13 | 1,62 | 64,8 | 300 | 0,004 | 1 | 50 | 8 | 8 | 55,3 | 92 | 8 | 70,2 | 94,3 | 66,7 |
| 14 | 1,42 | 56,8 | 235 | 0,0063 | 1,57 | 50 | 8 | 8 | 76,3 | 94 | 6 | 77,1 | 95,4 | 73,5 |
| 15 | 1,62 | 64,8 | 300 | 0,046 | 11,4 | 25 | 24 | 8 | 62,2 | 92 | 8 | 79,5 | 93,6 | 74,4 |
| 16 | 1,134 | 45,4 | 150 | 0,008 | 2 | 50 | 24 | 8 | 97,1 | 94 | 6 | 78,5 | 95,3 | 74,8 |

Exemples 17 à 19:

En opérant selon un mode opératoire similaire à celui décrit dans l'exemple 1 et sous des conditions réactionnelles mentionnées dans le tableau IV, on obtient au départ de l'alcool orthohydroxybenzylique OHB (saligénol) et de l'alcool vanillique, AV, les résultats indiqués dans le tableau IV.

TABLEAU IV

| Ex. N° | Nature de l'alcool | Poids de l'alcool | NaOH moles | g | Nature du catalyseur | Poids du catalyseur moles g | Eau en g | T 8°C | Durée t en heure | S% | T% | R% |
|---|---|---|---|---|---|---|---|---|---|---|---|---|
| 17 | OHB | 78,2g 0,63 mole | 2,88 | 115,2 | $CuSO_4,5H_2O$ | 0,046 11,4 | 300 | 50 | 24 | 84,1 | 100 | 84,1 |
| 18 | OHB | 78,2g 0,63 mole | 1,62 | 64,8 | $CuSO_4,5H_2O$ | 0,046 11,4 | 300 | 50 | 24 | 60,75 | 100 | 60,75 |
| 19 | AV | 97,1g 0,63 mole | 1,62 | 64,8 | $CuSO_4,5H_2O$ | 0,008 2 | 300 | 50 | 7 | | | 58,3 |

OHB: alcool orthohydroxybenzylique
AV: alcool vanillique

Il va de soi que la présente invention n'a été décrite qu'à titre purement explicatif et nullement limitatif et que toute modification utile pourra y être apportée sans sortir de son cadre tel que défini dans les revendications ci-après.

**Revendications**

1. Procédé pour la préparation d'hydroxybenzaldéhydes par oxydation d'alcools hydroxybenzyliques de formule générale

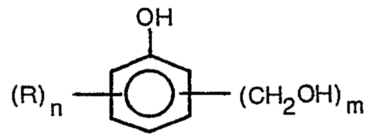
I

dans laquelle m est égal à 1 ou 2, n est égal à la différence 5-m et les restes R sont identiques ou différents et représentent chacun un atome d'hydrogène ou d'halogène ou un groupe alkyle, aryle, alcoxyle, hydroxyle, carboxyle ou méthylènedioxy ou un noyau condensé, avec l'oxygène ou de gaz contenant de l'oxygène caractérisé par le fait qu'il est effectué en milieu aqueux alcalin à une température comprise entre 25 et 50°C et en présence d'un hydroxyde alcalin en quantité telle que le rapport molaire hydroxyde alcalin/alcool hydroxybenzylique engagé soit compris entre 2,25 et 10.

2. Procédé selon la revendication 1 caractérisé par le fait qu'il est mis en oeuvre sous une pression d'oxygène cu de gaz contenant de l'oxygène supérieure ou égale à 1 bar.

3. Procédé selon l'une quelconque des revendications 1 et 2 caractérisé par le fait que l'hydroxyde alcalin utilisé est l'hydroxyde de sodium ou l'hydroxyde de potassium.

4. Procédé selon l'une quelconque des revendications 1 à 3 caractérisé par le fait qu'il est accéléré par addition d'un composé ou d'un mélange de composés d'un métal choisi parmi le cuivre, le cobalt, le fer et le manganèse.

5. Procédé selon l'une quelconque des revendications 1 à 4 caractérisé par le fait que le rapport molaire du composé métallique sur l'alcool hydroxybenzylique engagé est compris entre 0,01 et 0,1.

6. Procédé selon l'une quelconque des revendications 1 à 5 caractérisé par le fait qu'il est effectué en phase homogène avec une concentration pondérale en alcool hydroxybenzylique de départ compris entre 5 et 40%.

7. Procédé selon l'une quelconque des revendications 1 à 6 caractérisé par le fait qu'il est effectué en présence d'ions cuivriques ou d'ions cobalt II ou d'un mélange de ces ions.

8. Procédé selon l'une quelconque des revendications 1 à 7 caractérisé par le fait que l'on opère en présence d'un solvant organique inerte dans les conditions réactionnelles et miscible à l'eau.

9. Application du procédé selon l'une quelconque des revendications 1 à 8 à l'obtention du para hydroxybenzaldéhyde ou de l'aldéhyde salicylique ou un mélange de ces deux aldéhydes.

**Patentansprüche**

1. Verfahren zur Herstellung von Hydroxybenzaldehyden durch Oxydation von Hydroxybenzyl-Alkoholen der allgemeinen Formel

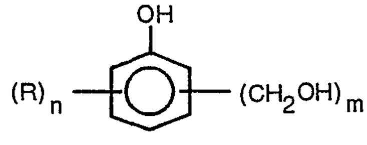
I

bei der m gleich 1 oder 2, n gleich der Differenz 5-m ist und die Reste R identisch oder unterschiedlich sein können und jeweils ein Wasserstoff- oder Halogen-Atom oder eine Alkyl-, Aryl-, Alcoxy-, Hydroxy-, Karboxyl-, oder Methylendioxy-Gruppe oder einen kondensierten Ring darstellen können, mit Sauerstoff oder einem sauerstoffhaltigen Gas, dadurch gekennzeichnet, daß das Verfahren in einer alkalischen wässrigen Umgebung bei einer Temperatur zwischen 25°C und 50°C und in Gegenwart eines Alkali-Hydroxydes in einer solchen Menge ausgeführt wird, daß das benutzte Molverhältnis Alkalihydroxyd/Hydroxybenzylalkohol zwischen 2,25 und 10 liegt.

2. Verfahren nach Anspruch 1, dadurch gekennzeichnet, daß es unter einem Druck des Sauerstoffes oder des sauerstoffhaltigen Gases ausgeführt wird, der größer oder gleich 1 bar ist.

3. Verfahren nach einem der Ansprüche 1 und 2, dadurch gekennzeichnet, daß das verwendete Alkali-Hydroxyd Natriumhydroxyd oder Kaliumhydroxyd ist.

4. Verfahren nach einem der Ansprüche 1 bis 3, dadurch gekennzeichnet, daß es durch Zugabe einer Verbindung oder eines Gemisches von Verbindungen eines Metalles beschleunigt wird, welches aus Kupfer, Kobalt, Eisen und Mangan ausgewählt ist.

5. Verfahren nach einem der Ansprüche 1 bis 4, dadurch gekennzeichnet, daß das verwendete Molverhältnis der Metallverbindung zu dem Hydrobenzylalkohol zwischen 0,01 und 0,1 liegt.

6. Verfahren nach einem der Ansprüche 1 bis 5, dadurch gekennzeichnet, daß es in homogener Phase mit einem Gewichtsanteil an Hydroxybenzylalkohol beim Beginn zwischen 5 und 40% ausgeführt wird.

7. Verfahren nach einem der Ansprüche 1 bis 6, dadurch gekennzeichnet, daß es in Anwesenheit von Cupri-Ionen oder von Kobalt (II) Ionen oder einem Gemisch dieser Ionen ausgeführt wird.

8. Verfahren nach einem der Ansprüche 1 bis 7, dadurch gekennzeichnet, daß man in Anwesenheit eines unter den Reaktionsbedingungen inerten und mit Wasser mischbaren organischen Lösungsmittels arbeitet.

9. Anwendung des Verfahrens nach einem der Ansprüche 1 bis 8 zur Erzielung von Parahydroxybenzaldehyd oder Salicyl-Aldehyd oder einem Gemisch der beiden Aldehyde.

**Claims**

1. A process for preparation of hydroxybenzaldehydes through oxidation of hydroxybenzylic alcohols of the general formula:

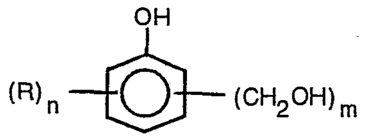

I

in which m is equal to 1 or 2, n is equal to the difference 5-m, and the remainders R are identical or different and each represent a hydrogen or halogen atom, or an alkyl, aryl, alkoxy, hydroxy, carboxy or methylenedioxy group, or a core condensed with oxygen or an oxygen-containing gas, characterized by conducting it in an alkaline aqueous medium at a temperature of between 25 and 50°C, and in the presence of an alkaline hydroxide in such a quantity that the molar ratio of alkaline hydroxide to hydroxybenzylic alcohol engaged therein is included between 2.25 and 10.

2. A process according to claim 1, characterized in that it is carried out under a pressure of oxygen or an oxygen-containing gas higher than, or equal to, 1 bar.

3. A process according to any one of claims 1 and 2, characterized in that the alkaline hydroxide used is sodium hydroxide or potassium hydroxide.

4. A process according to any one of claims 1, 2 and 3, characterized by accelerating it through addition of a compound or a mixture of compounds of a metal selected among copper, cobalt, iron and manganese.

5. A process according to any one of claims 1 through 4, characterized in that the molar ratio of the metallic compound to the hydroxybenzylic alcohol engaged therein is comprised between 0.01 and 0.1.

6. A process according to any one of claims 1 to 5, characterized in that it is conducted in homogeneous phase with a concentration by weight of starting hydroxybenzylic alcohol of between 5 and 40%.

7. A process according to any one of claims 1 to 6, characterized in that it is conducted in the presence of cupric ions or cobalt II-ions or a mixture of such ions.

8. A process according to any one of claims 1 to 7, characterized in that it is conducted in the presence of an organic solvent inert under the reactional conditions and miscible with water.

9. An application of the process according to any one of claims 1 to 8, for obtaining parahydroxybenzaldehyde or salicylic aldehyde or a mixture of both of these aldehydes.